Europäisches Patentamt

European Patent Office

Office européen des brevets

(19)

(11) Numéro de publication: **0 058 105**
**A2**

(12) **DEMANDE DE BREVET EUROPEEN**

(21) Numéro de dépôt: **82400093.9**

(22) Date de dépôt: **19.01.82**

(51) Int. Cl.³: **A 61 H 39/00**
**//A61N5/06**

(30) Priorité: **05.02.81 FR 8102792**

(43) Date de publication de la demande:
**18.08.82 Bulletin 82/33**

(84) Etats contractants désignés:
**AT DE FR GB IT LU NL SE**

(71) Demandeur: **Javelle, Edmond**
**Résidence Méridien A/21 20 Rue Gembloux**
**F-59240 Dunkerque(FR)**

(72) Inventeur: **Javelle, Edmond**
**Résidence Méridien A/21 20 Rue Gembloux**
**F-59240 Dunkerque(FR) -**

(74) Mandataire: **Ecrepont, Robert**
**Cabinet Ecrepont 12 Place Simon Vollant (Porte de Paris)**
**F-59800 Lille(FR)**

(54) **Ensemble régulateur de l'énergie circulant dans les méridiens.**

(57) L'invention se rapporte à ensemble régulateur de l'énergie circulant dans les méridiens.

Il est caractérisé en ce que le dit ensemble comprend deux appareils identiques, chacun conçu pour n'émettre la dite onde (13) qu'exclusivement à pleine puissance et sous deux fréquences d'impulsions ne variant entre elles que dans le rapport de un à deux.

Application à l'acupuncture.

Fig. 1

EP 0 058 105 A2

1

# ENSEMBLE REGULATEUR DE L'ENERGIE CIRCULANT DANS LES MERIDIENS

L'invention se rapporte à un ensemble régulateur de l'énergie circulant dans tous les méridiens du corps humain au moyen d'une onde infra-rouge découpée en tronçons émis à des fréquences prédéterminées, trouvant une entière application dans l'art de l'acupuncture et en ce sens, se substituant aux moyens classiques utilisés jusqu'alors : les aiguilles.

On connaît déjà des appareils émetteurs d'une onde infra-rouge découpée en tronçons émis à des fréquences prédéterminées (brevet français A-2279253).

Dans ces appareils, l'onde ainsi découpée est émise par une diode électroluminescente qui, en réponse à un train d'impulsions électriques, convertit cette énergie électrique en une onde électromagnétique qui, selon sa longueur, peut se situer dans le spectre infra-rouge (à compter de huit cents nanomètres).

Dans ces appareils connus, la diode utilisée est par exemple une diode à l'arseniure de gallium à laquelle les impulsions électriques sont fournies par un circuit oscillant.

Déterminant la fréquence de découpage de l'onde infra-rouge et la durée de chaque tronçon, la fréquence et la durée des impulsions électriques envoyées à la diode peuvent, à l'aide de circuits diviseurs et/ou de mise en forme de tout type connu, varier dans de larges proportions afin que l'onde infra-rouge puisse aussi bien être émise de manière pratiquement

continue qu'avoir lieu pendant des instants plus ou moins longs et plus ou moins espacés.

L'appareil objet de l'invention trouve exclusivement application en acupuncture classique, où à travers l'utilisation adéquate des points d'acupuncture des méridiens connus pour présenter non seulement une spécificité anatomique, histologique mais surtout une spécificité d'action, il permet donc de réguler l'énergie circulant dans tous les méridiens de ce corps humain, en respectant les grandes règles énergétiques qui sont différentes de l'auriculothérapie. L'appareil agira au niveau du point d'acupuncture dans le sens d'une stimulation ou encore d'une dispersion selon le but thérapeutique et la nature des troubles à traiter.

Parallèlement au traitement par l'acupuncture classique, selon laquelle on régule la circulation énergétique en agissant aux points précités répartis donc sur pratiquement tout le corps humain, s'est développé le traitement par l'auriculothérapie selon laquelle on traite certains troubles en agissant, non pas directement sur des trajets méridiens qui vont impacter des loges énergétiques d'importance majeure à l'intérieur de l'organisme, mais sur des points situés au niveau du pavillon de l'oreille où l'on retrouve les zones réflexes de chaque partie du corps, qu'il s'agisse d'un segment de membre, d'organes, de viscères, etc ...

Dans l'une et l'autre de ces méthodes, l'intervention se fait traditionnellement par des moyens entrant en contact matériel avec les points considérés.

Ce contact matériel traditionnel, consistait en des aiguilles constituant un handicap sérieux pour certaines utilisations que ce soit pour la douleur provoquée, pour la crainte de la piqûre, notamment de la part des enfants, ou pour le risque d'infection par perforation de la peau ou pour le risque de perforation de membranes séreuses, gaines nerveuses et vasculaires, etc ...

En auriculothérapie a déjà été utilisé un appareil de diagnostic et de soins substituant au contact matériel une simple projection sur une zone réflexe donnée d'une onde infra-rouge découpée selon une certaine fréquence pré-réglable (brevet français 2371935).

Chaque zone réflexe ne réagit normalement qu'à une fréquence de découpage qui lui est propre et cette réaction se manifeste sous la forme d'un signal vasculaire autonome décelable par une variation du pouls.

Pour l'établissement du diagnostic, à faible puissance afin de ne pas avoir d'effets traitants, l'onde est alors projetée sur une première zone à une fréquence de découpage, au départ quelconque, qu'on ajuste aussitôt jusqu'à l'apparition du signal vasculaire autonome.

En l'absence de réaction, on recommence l'opération avec une onde découpée selon une fréquence propre à une nouvelle zone que l'on projette sur les autres zones.

L'éventuelle apparition du signal vasculaire autonome dans l'une de ces autres zones qui donc réagirait à une fréquence qui pour elle serait "parasite" permettrait alors, par référence à la valeur de la fréquence et à la zone où la réaction apparaît anormalement, de diagnostiquer avec grande précision un trouble pour le traitement duquel la zone réflexe correspondante serait soumise également à une onde infra-rouge, mais dont, d'une part, la puissance aurait été portée cette fois à une valeur suffisante pour avoir des effets traitants et, d'autre part, le découpage aurait été fait non pas selon la fréquence normale pour la zone réflexe considérée mais selon la fréquence dite "parasite".

Un des inconvénients de cette méthode via les zones réflexes réside dans le fait que, outre la perte de temps non négligeable pour le praticien, il faut souvent recontrôler les zones de parasitage et recommencer.

D'aucuns ont pensé qu'il était possible de concevoir la projection de ces zones réflexes du pavillon de l'oreille au niveau de la surface du corps. Mais la pratique a révélé que cette transposition n'était pas valable et que même bien souvent la manipulation énergétique des méridiens avec de tels concepts aboutissait à des effets nuisibles ou contraires au but thérapeutique recherché.

En effet, celà s'est avéré incompatible avec la dynamique acupuncturale de manipulation de l'énergie au niveau des méridiens qui obéit à des lois propres bien définies dans le cadre des grandes lois énergétiques de l'acupuncture, selon l'inventeur.

Ainsi pour un méridien circulant sur un segment corporel précis, il n'y a pas correspondance à un niveau quelconque des différents points spécifiques d'acupuncture sélectionnés sur ce méridien et la somatotopie de ce segment sur la zone réflexe du pavillon de l'oreille en ce qui concerne le découpage de la fréquence de l'onde utilisée.

C'est ce que l'inventeur a découvert, mettant en évidence que des actions dangereuses, pouvant même aggraver les troubles, pourraient survenir de la transposition directe de la méthode d'auriculothérapie précédemment citée, à la manipulation énergétique au niveau des méridiens ou canaux énergétiques, où circulent pour chacun d'entre eux des énergies relativement spécifiques, porteuses de messages et de commandes précises pour tout l'organisme.

Il a donc remarqué qu'il était inutile de faire appel à des appareils émettant une onde sous plusieurs puissances car pour faire circuler l'énergie au niveau des méridiens il est inutile voire même dangereux de rechercher dans quelle zone réflexe fréquentielle des points on se trouve par corrélation avec la somatotopie au niveau de l'oreille.

En effet, agir autrement aboutit à des diffractions plus ou moins importantes, plus ou moins localisées de l'énergie circulant au sein des méridiens et par voie de conséquence soit à un effet thérapeutique insuffisant soit même à des nuisances parfois graves.

Il a de plus remarqué qu'il était erroné, contrairement aux enseignements donnés par l'état de la technique, de faire appel à des appareils émettant une onde découpée selon des fréquences qui, indépendamment de leurs valeurs réelles, sont de plus en plus nombreuses, et notamment de sept fréquences variant notamment dans un rapport de un à soixante quatre pour certains dispositifs à diode à arseniure de gallium (brevets français 2371935 et 2420352) et de seize fréquences variant dans un rapport de un à huit cents pour d'autres dispositifs à diode à effet laser (brevet français A-2390968).

Par ailleurs, les appareils utilisés en auriculothérapie s'avèrent de réglages délicats et possèdent des fréquences dangereuses dans leur application pratique : il faut rappeler que les fréquences de résonnance des différentes zones du pavillon de l'oreille sont très nombreuses et sont réparties dans de très larges proportions et plus précisément dans un rapport de un à soixante quatre.

L'appareil d'auriculothérapie doit donc offrir une plage de réglage de fréquence au moins aussi large, et le rayon infrarouge ainsi découpé doit de plus pouvoir être émis tant à faible qu'à forte puissance pour respectivement la recherche puis le traitement.

Ce qui n'a rien à voir avec l'objet de l'invention qui a permis de sélectionner seulement deux fréquences d'impulsions pour l'utilisation pratique en acupuncture, variant entre elles dans un rapport de un à deux.

L'une de ces fréquences sert à disperser l'énergie circulant dans les méridiens, l'autre à la stimuler ou la tonifier.

En effet, contrairement aux préjugés voulant que les points spécifiques des méridiens ne puissent réagir qu'aux fréquences variant dans de très larges proportions mises au point en auriculothérapie, l'inventeur a constaté que les règles de la circulation énergétique générale ne correspondaient pas à celles de l'auriculothérapie et a pu isoler dans la large plage évoquée plus haut les deux seules fréquences utiles et suffisantes pour traiter tous les troubles qui sont liés en fait à une perturbation de la circulation énergétique générale.

De surcroît, par cette méthode, l'efficacité du traitement était durable, l'utilisation des autres fréquences ayant au contraire un effet nul, insuffisant ou néfaste, provoquant par exemple une diffraction de l'énergie, d'où un gaspillage de cette précieuse énergie et un mauvais résultat.

Autre résultat que l'invention vise à obtenir est un ensemble peu encombrant, très simple à régler et répondant aux seules gammes de fréquences possibles dans la manipulation énergétique acupuncturale au niveau des méridiens, fréquences étudiées pour une efficacité maximale, rapide et non nuisible et tout à fait capables de remplacer les aiguilles classiques.

Par ailleurs, la diode a été soigneusement sélectionnée auprès de malades percevant les circuits méridiens à la moindre stimulation, ce qui a permis de sélectionner la plus efficace possible, ainsi que les seules fréquences d'utilisation possibles compte tenu de la rapidité d'obtention de résultats, de la rapidité plus ou moins marquée de la résolution de la symptomatologie selon le type de diode utilisée et le choix des fréquences assorti.

Le rayon émis par certaines d'entre elles peut également être mal toléré, or une stimulation en acupuncture ne doit jamais être désagréable.

Pour cette raison également la diode a été parfaitement sélectionnée, tenant compte des différences de profondeur de l'énergie en zones Yin et Yang.

Elle a également été choisie en fonction d'un certain diamètre permettant un certain degré d'action même lorsque le manipulateur se sera trompé au millimètre près dans la détermination anatomique ou topographique du point d'acupuncture dont la spécificité est capitale.

A cet effet, l'invention a pour objet un ensemble du type cité plus haut caractérisé en ce que le dit ensemble comprend deux appareils identiques chacun conçu pour n'émettre la dite onde qu'exclusivement à pleine puissance et sous deux fréquences d'impulsions ne variant entre elles que dans le rapport de un à deux.

L'invention sera bien comprise à l'aide de la description ci-après faite, à titre d'exemple non limitatif, en regard du dessin ci-annexé qui représente schématiquement :

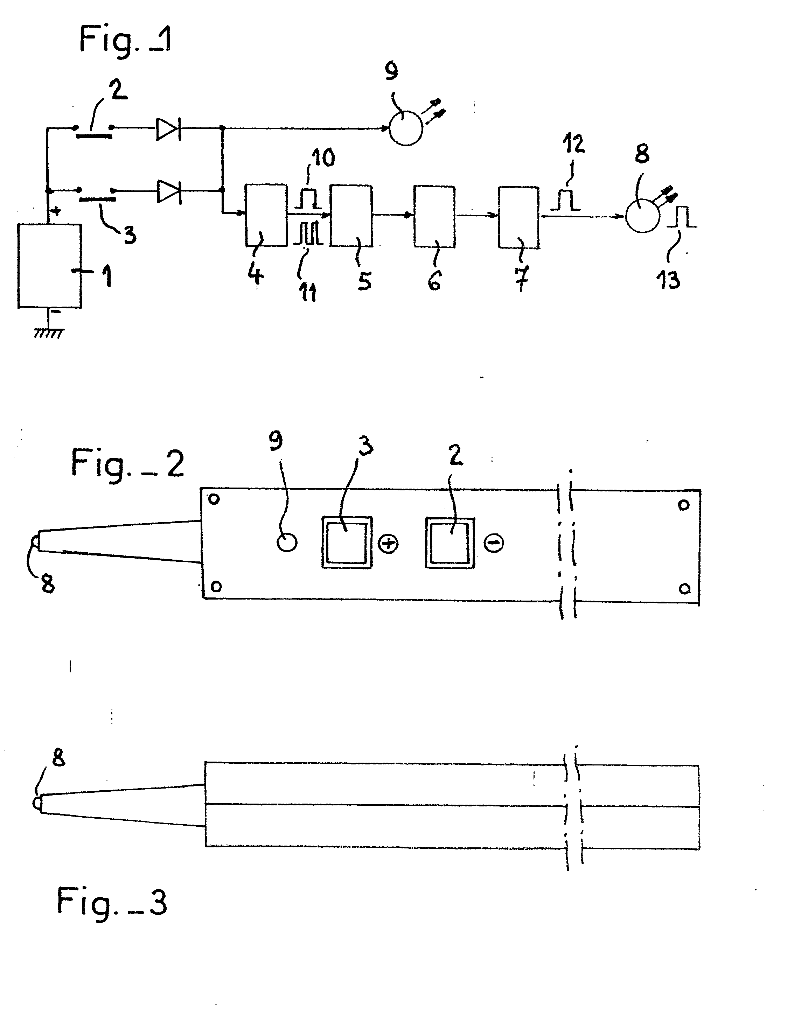

- figure 1 : le schéma de l'appareil,
- figures 2 et 3 : un appareil vu de face et de profil.

Comme indiqué plus haut, l'ensemble comprend deux appareils identiques équipés chacun d'une diode électroluminescente 8 (telle celle distribuée sous les références DEL : G a As EPITAXIE) dont la particularité est d'émettre un rayonnement 13 dans la bande infra-rouge et centrée sur neuf cent trente nanomètres (soit neuf mille trois cent angströms).

L'émission infra-rouge 13 sera rayonnée, par intermittence, à la fréquence dépendant de la touche de commande 2 ou 3 choisie, à savoir une touche 2 dite de dispersion et une touche 3 dite de tonification, lesquelles font fonctionner l'oscillateur 4 à des fréquences de base 10-11 différentes du fait de la mise en service de réseaux "résistance-condensateur" différents, déterminés de manière que les dites fréquences de base soient l'une par rapport à l'autre dans un rapport de un à deux.

La fréquence de base 10 correspondant à la touche de dispersion 2 est de quatre mille six cent cinquante hertz plus ou moins dix pour cent, et la fréquence de base 11 correspondant à la touche de dispersion 3 est de neuf mille trois cents Hertz plus ou moins dix pour cent, lesquelles fréquences sont selon l'inventeur les seules utilisables en acupuncture.

Un transistor 5 permet de calibrer les impulsions 10 ou 11 avant de les diviser par un circuit diviseur 6.

Après le diviseur, un différenciateur 7, comprenant principalement un transistor, permet de calibrer correctement le découpage aux fréquences désirées avec le rapport cyclique adéquat.

Par des impulsions électriques 12, la diode 8 est donc alimentée :
- en dispersion, toutes les mille sept cent vingt nanosecondes,
- en tonification, toutes les huit cent soixante nanosecondes,
et la durée de conduction de la diode est de quarante nanosecondes.

Toutes ces valeurs peuvent évidemment varier de plus ou moins dix pour cent.

Un voyant 9, constitué par une diode électroluminescente émettant dans le spectre visible, indique la mise sous tension de la pile 1.

L'intensité lumineuse de ce voyant 9 donne, quant à elle, une appréciation de l'état d'usure de cette pile 1.

L'ensemble comprend également un appareil récepteur du rayonnement infra-rouge 13 afin de capter et de contrôler avec précision l'état d'usure de la pile 1 et la qualité d'émission de la diode infra-rouge 8.

Dans le but de parvenir à un bon équilibre énergétique de l'individu, de bien réguler partout les besoins et désirs de son organisme, de manière caractéristique, l'appareil ne peut émettre que sous deux fréquences d'impulsion :

- l'une pour disperser l'énergie, pour l'empêcher de poursuivre son chemin et prendre éventuellement une voie de dérivation,

- l'autre pour la stimuler, pour l'inviter à poursuivre son cheminement habituel plus rapidement, ou encore pour marquer davantage l'action physiologique du point au sein du méridien.

L'inventeur à ce sujet insiste sur une notion capitale d'utilisation courante en acupuncture : la notion de point inducteur et de point directeur.

En effet, l'énergie empruntant de nombreux méridiens tels de véritables réseaux électroniques, il en résulte de nombreux points carrefours et de nombreux points possédant des caractéristiques physiologiques multiples ; il faudra donc guider l'énergie par les points de commande, par les points carrefours car sinon parvenue à ces endroits, l'énergie ne saura plus où aller.

Il faudra donc lui faire comprendre ce que l'on attend d'elle d'où l'utilisation alors simultanée de deux points répondant à cette notion de point inducteur et de point directeur, notion extrêment importante en application pratique.

D'où, à cet effet, l'utilisation nécessaire de tous les instants d'un ensemble formé de deux appareils, non seulement pour répondre à cette notion mais également de façon à agir de manière symétrique sur l'énergie du corps, à bien équilibrer cette énergie.

Ainsi, avant tout traitement en acupuncture est-il nécessaire d'opérer une rééquilibration énergétique générale rendant obligatoire la manipulation simultanée et symétrique de deux appareils au niveau des points d'acupuncture.

La démonstration est donc ainsi faite que cet ensemble d'appareil est conçu de façon bien différente quant à ses applications atiques, de l'auriculothérapie qui répond à d'autres règles.

Il trou son application pratique, immédiate, extrêmement efficace, à tous les âges, totalement indolore, et sans aucune nuisance de par le choix précis des fréquences que l'on peut à la limite envisager d'élargir à dix pour cent près.

Il est bien évident que l'invention n'est pas limitée à ce qui est ci-dessus décrit et qu'elle peut comprendre de nombreuses variantes de réalisation, ne serait-ce qu'en ce qui concerne l'aspect extérieur et tant la diode utilisée et avec elle les paramètres caractérisant les impulsions que sont leur fréquence, leur amplitude et leur durée, qu'en ce qui concerne le mode de transmission de l'onde, soit directement tels lasers ou diodes laser, soit par l'intermédiaire de conducteurs de lumière tels des fibres optiques.

REVENDICATIONS

1. Ensemble régulateur de l'énergie circulant dans tous les méridiens du corps humain, et ce, selon les troubles à traiter, dans le sens d'une dispersion ou d'une stimulation de la dite énergie, au moyen d'une onde émise par une diode (8) lorsque celle-ci reçoit des impulsions électriques (12) qui lui sont envoyées par un circuit électrique (4, 5, 6, 7) C A R A C T E R I S E en ce que le dit ensemble comprend deux appareils identiques, chacun conçu pour n'émettre la dite onde (13) qu'exclusivement à pleine puissance et sous deux fréquences d'impulsions ne variant entre elles que dans le rapport de un à deux.

2. Ensemble selon la revendication 1 caractérisé en ce que les impulsions (12) sont sous la fréquence de dispersion envoyée toutes les mille sept cent vingt nanosecondes et sous la fréquence de stimulation envoyée toutes les huit cent soixante nanosecondes à plus ou moins dix pour cent.

3. Ensemble selon l'une quelconque des revendications 1 et 2, caractérisé en ce qu'il comprend un moyen captant et contrôlant l'onde émise.

Fig. _1

Fig. _2

Fig. _3